(19) Europäisches Patentamt
European Patent Office
Office européen des brevets

(11) Publication number: **0 168 013**
**B1**

(12) **EUROPEAN PATENT SPECIFICATION**

(45) Date of publication of patent specification: **02.03.88**

(51) Int. Cl.[4]: **C 07 C 93/10,** C 07 C 149/42, A 61 K 31/135

(21) Application number: **85108443.4**

(22) Date of filing: **08.07.85**

(54) **Fluoroallylamine derivatives.**

(30) Priority: **13.07.84 US 630556**

(43) Date of publication of application:
**15.01.86 Bulletin 86/03**

(45) Publication of the grant of the patent:
**02.03.88 Bulletin 88/09**

(84) Designated Contracting States:
**AT BE CH DE FR IT LI LU NL SE**

(56) References cited:
**DE-A-1 593 800**
**FR-M- 3 075**
**GB-A-1 110 378**
**US-A-3 221 054**
**PSYCHOPHARMACOLOGY, vol. 62, 1979 S. LIPPER et al. "Comparative behavioral effects of clorgyline and pargyline in man; a preliminary evaluation" pages 123-128**
**TRENTS IN NEURO SCIENCES, vol. 2, no5, 1979 J. KNOLL "(-)Deprenyl- the MAOinhibitor without the cheese effect." pages 111-113**

(73) Proprietor: **MERRELL DOW PHARMACEUTICALS INC.**
**2110 E. Galbraith Road**
**Cincinnati Ohio 45215 (US)**

(72) Inventor: **McDonald, Ian Alexander**
**13 Rue des Lilas Pfulgriesheim**
**F-67370 Truchtersheim (FR)**

(74) Representative: **Sgarbi, Renato et al**
**Gruppo Lepetit S.p.A. Patent & Trademark Department 34, Via Roberto Lepetit**
**I-21040 Gerenzano (Varese) (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 168 013 B1

**Description**

This invention relates to novel chemical compounds, to their use as medicaments and to pharmaceutical compositions employing said compounds.

The class of compounds known as monoamine oxidase inhibitors (MAO inhibitors) has been employed in psychiatry for over 20 years for the treatment of depression [See Goodman and Gilman, *The Pharmacological Basis of Therapeutics,* 6th Ed., McMillan Publishing Co., Inc., N.Y., 1980, pages 427—430]. MAO Inhibitors currently used in the USA for treating depression are tranylcypromine (PARNATE, SKF), phenelzine (NARDIL, Parke-Davis), and isocarboxazid (MARPLAN, Roche). In addition, another MAO inhibitor, pargyline (EUTRON, Abbott), is available for the treatment of hypertension [See *Physicians' Desk Reference,* 34th Ed., Medical Economics Co., Oradell, M.J. 1980, pages 1327—1328 (phenelzine), pages 1466—1468 (isocarboxazid), pages 1628—1630 (tranylcypromine), and pages 521—522 (pargyline)]. In addition to being used in treating depression, MAO inhibitors can be employed to treat other psychiatric disorders, such as phobic anxiety states.

It is believed that the MAO inhibitors act to alleviate psychiatric disorders, such as depression, by increasing the concentration of one or more biogenic monoamines in the brain or sympathetic nervous system. The monoamine oxidase enzyme (MAO) plays an important role in the metabolic regulation of the monoamines since it catalyzes the biodegredation of the monoamines through oxidative deamination. By inhibiting MAO, the degradation of the monoamines is blocked, and the result is an increase in the availability of the monoamines for their physiological functions. Among the physiologically active monoamines which are known substrates for MAO are: (a) the so-called "neurotransmitter" monoamines, such as the catecholamines (e.g. dopamine, epinephrine, and norepinephrine) and the indoleamines (e.g. tryptamine and 5-hydroxytryptamine), (b) the so-called "trace" amines (e.g. *o*-tyramine, phenethylamine, *tele-N*-methylhistamine), and (c) tyramine.

The usefulness of the MAO inhibitors in treating depression is limited because the administration of such agents can potentiate the pharmacological actions of certain food substances or drugs leading to dangerous and sometimes lethal effects. For example, persons receiving a MAO inhibitor must avoid the ingestion of foods which have a high tyramine content (such as cheese) because the MAO inhibitor will block the metabolic degradation of tyramine in the gut to produce high circulating levels of tyramine, consequent release of catecholamines in the periphery, and finally serious hypertension. The potentation by a MAO inhibitor of the pressor effect of tyramine arising from the ingestion of cheese, and the hypertensive episode produced thereby, are commonly known as the "cheese reaction" or "cheese effect". Moreover, persons on conventional MAO therapy cannot be given directly-acting sympathomimetic drugs (or precursors thereof) which are themselves substrates for MAO (e.g. dopamine, epinephrine, norepinephrine, or L—DOPA) or indirectly-acting sympathomimetic drugs (e.g. amphetamines or cold, hay-fever, or weight control preparations that contain a vasoconstrictor). The potentiation of the pressor effect of indirectly-acting sympathomimetic drugs is especially profound. This is because such drugs act peripherally primarily be releasing catecholamines in nerve endings, and the concentration of the liberated catecholamines will be dangerously elevated if the metabolic degradation of the catecholamines via MAO is blocked. In addition, a MAO inhibitor should not be used in combination with another MAO inhibitor or with hypotensive agents, dibenzazepine antidepressants, meperidine, CNS depressants, and anticholinergic agents.

Biochemical and pharmacological studies indicate that the MAO enzyme exists in two forms known as "MAO Type A" (MAO—A) and "MAO Type B" (MAO—B). The two forms differ in their distribution in body organs, in their substrate specificity, and in their sensitivity to inhibitors. In general. MAO—A selectively oxidizes the so-called "neurotransmitter" monoamines (epinephrine, norepinephrine, and 5-hydroxytryptamine) while MAO—B selectively oxidizes the "trace" monoamines (*o*-tyramine, phenethylamine, and *tele-N*-methylhistamine). Both MAO—A and MAO—B oxidize tyramine, tryptamine, and dopamine. However, in man, dopamine has been shown to be a preferred substrate for MAO—B. The forms also differ in their sensitivity to inhibition, and thus they can be preferentially inhibited depending upon the chemical structure of the inhibitor and/or the relative concentrations of the inhibitor and the enzyme. The MAO inhibitors currently sold in the USA for the therapy of depression (tranylcypromine, phenelzine, and isocarboxazid) are not preferential in their action upon MAO. However, various chemical compounds are known in the art to be preferential inhibitors of MAO, the most important being clorgyline, pargyline, and L-deprenyl which are all reported to be clinically effective antidepressant agents. MAO—A is preferentially inhibited by clorgyline, while MAO—B is preferentially inhibited by pargyline and L-deprenyl. It should be observed that the "selectivity" of a MAO inhibitor arises because the inhibitor has a greater affinity for one form of the enzyme. Thus, the selectivity of an inhibitor for MAO—A or MAO—B *in vivo* will be dose-dependent, selectivity being lost as the dosage is increased. Clorgyline, pargyline, and L-deprenyl are selective inhibitors at lower dosages, but are not selective inhibitors at higher dosages. The literature concerning MAO—A and MAO—B and the selective inhibition thereof is extensive [See, for example, Goodman and Gilman, *ibid,* pages 204—205; Neff *et al., Life Sciences, 14,* 2061 (1974); Murphy, *Biochemical Pharmacology, 27,* 1889 (1978); Knoll, Chapter 10, pages 151—171 and Sandler, Chapter 11, pages 173—181, in *Enzyme Inhibitors as Drugs,* M. Sandler, Ed., McMillan Press Ltd., London, 1980; Lipper *et al., Psychopharmacology, 62,* 123 (1979); Mann *et al., Life Sciences, 26,* 877 (1980); and various articles in

*Monoamines Oxidase: Structure, Function, and Altered Functions,* T. Singer *et al.* Ed., Academic Press, N.Y., 1979].

Of the selective inhibitors of MAO, L-deprenyl is of interest since the "cheese effect" is not observed at the low dosages where preferential inhibition of MAO—B occurs (See Knoll, *TINS,* pages 111—113, May 1979]. This observation is not unexpected since the intestinal mucosa contains predominantly MAO—A which, because it is not inhibited, permits oxidation and removal of the ingested tyramine. The selectivity of L-deprenyl for MAO—B may account for its ability to potentiate L—DOPA for the treatment of Parkinson's disease without producing peripheral side effects, such as hypertension due to potentiation of pressor catecholamines [See Lees *et al., Lancet,* pages 791—795, October 15, 1977 and Birkmeyer, *Lancet,* pages 439—443, February 26, 1977].

In its first composition of matter aspect, this invention comprehends pharmacologically active fluoroallylamine derivatives of the following formula I:-

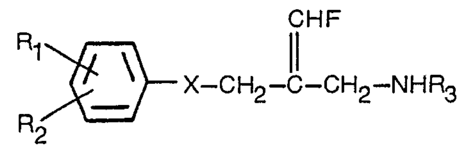

(I)

wherein:-

$R_1$ and $R_2$ independently represent hydrogen, chlorine or fluorine;

$R_3$ represents hydrogen or ($C_1$—$C_4$) alkyl; and

X represents oxygen or sulfur, and pharmacologically acceptable acid addition salts thereof.

The compounds of Formula I are pharmacologically active, being capable of inhibiting MAO *in vitro* and *in vivo.* They are useful for the treatment of psychiatric disorders, in particular depression, which are known to be responsive to MAO inhibitor therapy. For the treatment of depression, the compounds can be employed in a manner similar to that of the known clinically active MAO inhibitors, such as phenelzine and tranylcypromine.

Surprisingly, the compounds of Formula I are capable of preferentially inhibiting the B form of MAO *in vitro* and, at suitable low dosages *in vivo,* such compounds will inhibit MAO—B without substantially inhibiting MAO—A. At dosage levels where such compounds exert a selective effect on MAO—B, the compounds will not produce a marked "cheese effect". Hence, as with L-deprenyl, a known selective inhibitor of MAO—B, such compounds can be employed at suitable dosages for the treatment of depression, or for the potentiation of L—DOPA in the treatment of Parkinsonism, with a significantly decreased risk of producing side effects, such as the "cheese effect". The preferred compounds of Formula I showing selective inhibition of MAO—B are 2-phenoxymethyl-3-fluoroallylamine, 2-thiophenoxymethyl-3-fluoroallylamine and especially 2-(2',4'-dichlorophenoxy)methyl-3-fluoroallylamine. These compounds, therefore, are the most preferred embodiments of Formula I.

As employed herein, the term "alkyl" contemplates both straight- and branched-chain alkyl groups. Straight-chain alkyl groups are preferred. Illustrative examples of ($C_1$—$C_4$) alkyl groups are methyl, ethyl, *n*-propyl, *iso*-propyl, *n*-butyl, *iso*-butyl, and *tert*-butyl. Methyl and ethyl are the most preferred alkyl groups.

When one or both of $R_1$ and $R_2$ is other than hydrogen, the relevant substituent group can be located at any of the available positions in the phenyl ring (i.e. in the *ortho, para,* or *meta* positions). When the phenyl ring is substituted by two substituent groups, the groups can be different but preferably are the same. Presently, 2,4 disubstitution is preferred.

It will be apparent to those skilled in the art that, because the compounds of Formula I contain a double bond, geometric isomerism is possible. It should be understood, therefore, that in Formula I, the fluorine atom can be oriented in the *cis* position or in the *trans* position. In naming compounds of Formula I herein, the prefixes "(E)" and "(Z)" are used in the conventional manner to indicate the stereochemistry at the double bond. If no stereochemical designation is given, both the substantially pure isomers, or mixtures thereof, are meant.

Presently preferred compounds of Formula I are those in which $R_3$ represents hydrogen. The more preferred compounds of Formula I are those wherein $R_3$ represents hydrogen and $R_1$ and $R_2$ independently represent hydrogen or chlorine. It is also preferred that X represents oxygen.

Illustrative examples of the compounds of Formula I are:

2-(2'-chlorophenoxy)methyl-3-fluoroallylamine,
2-(4'-chlorophenoxy)methyl-3-fluoroallylamine,
2-(4'-fluorophenoxy)methyl-3-fluoroallylamine,
2-thiophenoxymethyl-3-fluoroallylamine
2-(2',4'-dichlorophenoxy)methyl-3-fluoroallylamine,
2-(2',4'-dichlorothiophenoxy)methyl-3-fluoroallylamine,
2-(5'-chloro-3'-fluorophenoxy)methyl-3-fluoroallylamine,
2-(2'chlorothiophenoxy)methyl-3-fluoroallylamine,
2-(4'-fluorothiophenoxy)methyl-3-fluoroallylamine,
2-phenoxymethyl-3-fluoroallylamine,
2-(2'-chloro-4-'-fluorothiophenoxy)methyl-3-fluoroallylamine,

A further object of the present invention is represented by a compound of formula I for use as a medicine, and in particular, for use in the treatment of depression. To this aim, an effective amount of a compound of formula I or a pharmacologically acceptable acid addition salt thereof is given to a depressed patient.

For pharmacological use, the compounds of Formula I may be administered in the form of an acid addition salt of a non-toxic organic or inorganic acid. Appropriate salts are those formed, for example, from the following acids: hydrochloric, hydrobromic, sulfonic, sulfuric, phosphoric, nitric, maleic, fumaric, benzoic, ascorbic, pamoic, succinic, methanesulfonic, acetic, propionic, tartaric, citric, lactic, malic, mandelic, cinnamic, palmitic, itaconic, and benzenesulfonic.

When employed to treat depression, the effective dosage of the compounds of Formula I will vary according to the particular compound being employed, the severity and nature of the depression and the particular subject being treated. In general, effective results can be achieved by administering a compound at a dosage level from about 5mg to about 100 mg per day, given systemically. Therapy should be initiated at lower dosages, the dosage thereafter being increased until the desired effect is obtained.

At dosage levels set forth above, the compounds of Formula I will, in general, inhibit both forms of MAO. However, at lower dosage levels, they will preferentially inhibit MAO—B and have a decreased risk of producing the "cheese effect". Thus, for example, 2-(2'4'-dichlorophenoxy)methyl-3-fluoroallylamine, 2-phenoxymethyl-3-fluoroallylamine, or 2-thiophenoxy-3-fluoroallylamine will selectively inhibit MAO—B at a systemic dosage range of about 0.1 mg to about 5 mg per day. At this dosage range, the risk of adverse reaction from the "cheese effect" will be substantially reduced or eliminated.

The active compounds of this invention can be administered in various manners to achieve the desired effect. The compounds can be administered alone or in combination with pharmaceutically acceptable carriers or diluents, the proportion and nature of which are determined by the solubility and chemical properties of the compound selected, the chosen route of administration, and standard pharmaceutical practice. The compounds may be administered orally in solid dosage forms, e.g. capsules, tablets, powders, or in liquid forms, e.g. solutions or suspensions. The compounds may also be injected parenterally in the form of sterile solutions or suspensions. Solid oral forms may contain conventional excipients, for instance: lactose, succrose, magnesium stearate, resins, and like materials. Liquid oral forms may contain various flavoring, coloring, preserving, stablizing, solubilizing, or suspending agents. Parenteral preparations are sterile aqueous or nonaqueous solutions or suspensions which may contain certain various preserving, stabilizing, buffering, solubilizing, or suspending agents. If desired, additives, such as saline or glucose may be added to make the solutions isotonic.

The amount of active compound administered will vary and can be any effective amount. Unit doses of these compounds can contain, for example, from about 5 mg to about 100 mg of the compounds and may be administered, for example, one or more times daily, as needed.

The term "unit dosage form" is used herein to mean a single or multiple dose form containing a quantity of the active ingredient in admixture with or otherwise in association with the diluent or carrier, said quantity being such that one or more predetermined units are normally required for a single therapeutic administration. In the case of multiple dose forms such as liquids or scored tablets, said predetermined unit will be one fraction such as 5 ml (teaspoon) quantity of a liquid or a half or quarter of a scored tablet, of the multiple dose form.

In the composition aspect of the invention, there are provided pharmaceutical formulations in which form the active compounds of the invention will normally be utilized. Such formulations are prepared in a manner well known *per se* in the pharmaceutical art and usually comprise at least one active compound of the invention in admixture or otherwise in association with a pharmaceutically acceptable carrier or diluent therefore. A carrier or diluent may be solid, semi-solid, or liquid material which serves as a vehicle, excipient, or medium for the active ingredient. Suitable diluents or carriers are well known *per se.* The pharmaceutical formulations may be adapted for enteral or parenteral use and may be administered to the patient in the form of tablets, capsules, suppositories, solutions, suspensions, or the like.

In the specific Examples included hereinbelow, illustrative examples of suitable pharmaceutical formulations are described.

The compounds of Formula I in which $R_3$ represents hydrogen can be obtained by reaction in manner known *per se* between an amino-protected derivative of the corresponding 1-fluoro-2-bromomethyl-3-aminopropene of the following Formula II and the corresponding phenol or thiophenol of the following Formula III and subsequent removal of the amino protecting group.

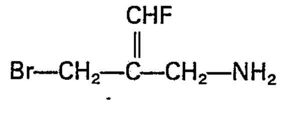 (II)

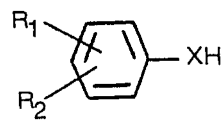 (III)

In Formulae II and III, $R_1$ and $R_2$ are as defined in connection with Formula I. The reaction is carried out under anhydrous conditions in the presence of a strong base, especially sodium hydride or butyl lithium, in an aprotic solvent, especially tetrahydrofuran. Usually, the reaction will proceed at room temperature.

Both amino hydrogen atoms of the 1-fluoro-2-bromomethyl-3-aminopropene must be protected during reaction with the phenol or thiophenol. Preferably, the protecting group is phthaloyl and

conveniently the 1-fluoro-2-bromomethyl-3-phthalimidopropene is prepared directly by bromination in manner known *per se* of the corresponding 1-phthalimido-2-methyl-3-fluoro-2-propene of the following Formula IV:

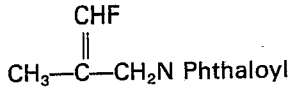

(IV)

Conveniently, the bromination is carried out using N-bromosuccinimide as the brominating agent.

The compounds of Formula IV can be obtained in manner known *per se* by treating the corresponding 2-methyl-3-fluoroallyl alcohol of the following Formula V with phthalimide in the presence of a triarylphosphine or trialkylphosphine and diethyl azodicarboxylate in an aprotic solvent, especially tetrahydrofuran or dioxane.

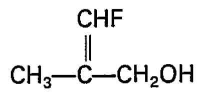

(V)

The compounds of Formula V can be obtained in manner known *per se* by reduction of the corresponding ethyl 2-methyl-3-fluoroacrylate of the following Formula VI

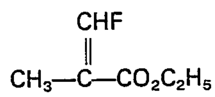

(VI)

Suitably, the reducing agent employed is diisobutylaluminium hydride in hexane, tetrahydrofuran, diethyl ether or dichloromethane or mixtures thereof at a reaction temperature of 0 to −75°C.

The compounds of Formula VI can be obtained in manner known *per se* by selectively hydrolyzing the t-butyl ester group of the corresponding t-butyl 2-difluoromethyl-2-carbethoxyalkonate of the following Formula VII and subsequently decarboxylating the resultant 2-difluoromethyl-2-carbethoxyalkanoic acid of the following Formula VIII by treatment with a base

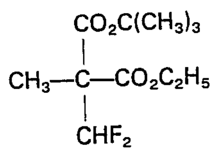

(VII)

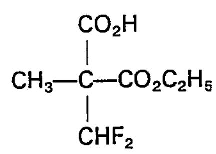

(VIII)

Suitably, the selective hydrolysis is carried out by treatment with an acid, preferably trifluoroacetic acid. The decarboxylation also eliminates one of the two fluorine atoms of the difluoromethyl moiety to provide the required acrylate of Formula VI. Suitably, a weak base, such as sodium bicarbonate, is employed to prevent excess base reacting with the double bond.

The compounds of Formula VII can be prepared by conventional difluoromethylation of the corresponding t-butyl 2-carbethoxyalkanoate (which are known *per se*) using sodium tert-butoxide and reacting the resultant carbanion with chlorodifluoromethane.

The amino-protected product of the reaction between the amino-protected derivative of the compound of Formula II and the phenol or thiophenol of Formula III is converted into the required compound of Formula I by removal of the protecting group in manner known *per se.* When the protecting group is phthaloyl, the said product can be cleaved by heating with hydrazine in an organic solvent or by heating with a strong mineral acid or a mixture of hydrochloric and acetic acids.

Compounds of Formula I in which $R_3$ represents alkyl can be prepared from the corresponding primary amines of Formula I (i.e. $R_3$ represents hydrogen) by conventional N-alkylation methods. For example, N-ethyl derivatives ($R_3$ represents ethyl) can be obtained by treating the primary amine with benzaldehyde in a lower alcohol, e.g. ethanol, to form a Schiff's base, treating the Schiff's base with triethyloxonium tetrafluoroborate, and hydrolyzing the intermediate thus formed.

The compounds produced by the foregoing processes may be isolated either *per se* or as acid addition salts thereof.

The acid addition salts are preferably the pharmaceutically acceptable, non-toxic addition salts with suitable acids such as those previously referred to in this Specification. Apart from pharmaceutically acceptable acid addition salts, other salts are also included within the scope of acid addition salts, such as for example, those with picric or oxalic acid; they may serve as intermediates in the purification of the compounds or in the preparation of other, for example, pharmaceutically acceptable, acid addition salts, or are useful for identification or characterisation of the base.

A resulting acid addition salt may be converted into the free compound according to known methods, for example, by treating it with an alkali or alkaline earth metal hydroxide or alkoxide; with an alkali metal

or an alkaline earth metal carbonate or hydrogen carbonate; with trialkylamine; or with an anion exchange resin.

A resulting acid addition salt may also be converted into another acid addition salt according to known methods; for example, a salt with an inorganic acid may be treated with sodium, barium or silver salt of an acid in a suitable diluent, in which a resulting inorganic salt is insoluble and is thus removed from the reaction medium. An acid addition salt may also be converted into another acid addition salt by treatment with an anion exchange preparation.

The invention is illustrated in the following non-limiting Examples in which all temperatures are specified in degrees Centigrade.

Example 1

(Z)-2-(2',4'-dichlorophenoxy)methyl-3-fluoroallylamine

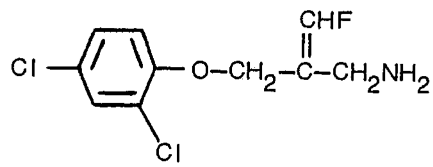

A. tert-Butyl 2-Carbethoxypropionate

A solution of diethyl methylmalonate (500 g) in ethanol (1000 ml) is treated with a clear solution of potassium hydroxide (116 g) in ethanol (1500 ml) for 16 hours. The mixture is concentrated to 1500 ml, filtered, then kept at −20°C overnight by which time colorless needles form. These are filtered and dried to give a colorless product (335 g).

This product is dissolved in water (145 ml), cooled to about 5°C and treated with concentrated hydrochloric acid (161 ml). After 1 hour, water is added and the product (254 g, 60% yield, colorless liquid) is isolated by ether extraction.

A portion (234 g) of this product is dissolved in anhydrous ether (600 ml), cooled in acetone-dry ice bath and treated consecutively with sulfuric acid (15 ml) and liquid isobutylene (600 ml). The reaction flask is firmly stoppered, the cooling removed and the solution is stirred for 6 hours. The solution is again cooled and treated with isobutylene (600 ml), then the reaction is left overnight. The solution is then poured into water (200 ml) containing potassium carbonate (115 g) and the mixture is extracted with ether. The ether extract gives *tert-butyl 2-carbethoxypropionate* (218 g, 67% yield) as a colorless liquid; b.p. 70°C (oven)/ 0.05 mm Hg.

NMR ($CCl_4$): δ 1.24, t (J=7Hz), 3H; 1.41, m, 12H; 3.17, q (J=7Hz), 1H; 4.13, q (J=7Hz), 2H.

B. tert-Butyl 2-Difluoromethyl-2-carbethoxypropionate

A slurry of sodium *tert*-butoxide (32.91 g) in dry tetrahydrofuran (THF) (200 ml) is stirred while a solution of *tert*-butyl 2-carbethoxypropionate (34.62 g) (prepared in Step A) in THF (100 ml) is added at a fast drop rate. The mixture is heated to 45°C then the clear solution is treated with a fast stream of Freon 22 (Trade Mark) for about 5 min. The temperature rises rapidly and then falls at which time the Freon 22 addition is stopped. The heating bath is removed and the mixture is stirred for 1 hour, ice is added to reduce the temperature about 20°C, then the mixture is washed several times with water. To ensure good separation of the layers, ether and small amounts of dilute aqueous hydrochloric acid are added when necessary. After drying the organic layer over magnesium sulfate the solvents are evaporated to leave a pale, orange oil (39.61 g, 92% yield). Generally, this material is sufficiently pure for the next step. Distillation of a small portion gives *tert butyl 2-difluoromethyl-2-carbethoxypropionate* as a colorless liquid; b.p. 90°C (oven), 0.05 mm. Hg.

NMR ($CCl_4$): δ 1.26, t (J=7Hz), 3H; 1.42, s, 12H; 4.19, q (J=7Hz), 2H; 6.20, t (J=56Hz), 1H.

Analysis for $C_{11}H_{18}F_2O_4$

| | | |
|---|---|---|
| Found: | C, 52.47; | H, 7.07% |
| Requires: | C, 52.37; | H, 7.19% |

C. (E)-Ethyl 2-Methyl-3-fluoroacrylate

A solution of *tert*-butyl 2-difluoromethyl-2-carbethoxypropionate (392 g) (prepared in Step B) in trifluoroacetic acid (TFA) (400 ml) is stirred at room temperature for several hours, then the excess TFA is removed by evaporation at reduced pressure. The residue is treated with carbon tetrachloride and reevaporated.

The product is divided into two and each part is treated as follows:

A mixture of the crude acid, water (200 ml), chloroform (2000 ml) and sodium bicarbonate (250 g) is refluxed (bath temperature 70°C) and vigorously stirred for 5.5 hours, then the mixture is allowed to cool to

room temperature. The chloroform layer is separated, dried (magnesium sulfate), filtered and fractionally distilled at atmospheric pressure. The material with a boiling point range of 70—115°C was redistilled at reduced pressure to give essentially pure *(E)-ethyl 2-methyl-3-fluoroacrylate* (45 g, 22% yield) as a colorless liquid; b.p. 60—70°C/80 mm. Hg.

NMR ($CCl_4$): δ 1.25, t (J=7Hz), 3H; 1.79, d.d (J=4Hz, 1.5Hz), 3H; 4.13 t (J=7Hz), 2H; 7.48, d.m (J=86Hz), 1H.

D. (E)-2-Methyl-3-fluoroallyl Alcohol

A solution of diisobutylaluminium hydride (1318 ml) is added during 30 min to THF (1000 ml) cooled to −55 to −65°C, then a solution of (*E*)-ethyl 2-methyl-3-fluoroacrylate (58 g) (prepared in Step C) in THF (50 ml) is added over 15 min. The cooling bath is removed and the temperature is allowed to rise to 18°C during 3 hours. Using an ice-salt bath, the solution is cooled and methanol (107 ml) is added so that the temperature is in the range −10° to +5°C, then after an additional 30 min water (175 ml) is added with the temperature at −5°C to +5°C. The cooling is removed and the mixture is stirred for 1 hour and filtered. The filtrate is dried (magnesium sulfate), filtered and fractionally distilled at atmospheric pressure at first, then under reduced pressure. In this way pure *(E)-2-methyl-3-fluoroallyl alcohol* is obtained as a colorless liquid (19.0 g, 48% yield); b.p. 63°C/37 mm. Hg.

NMR ($CDCl_3$): δ 1.71, d.d (J =3Hz, 1.5Hz), 3H; 2.07, s, 1H; 3.98, d.d (J=4Hz, 0.8Hz), 2H; 6.60, d.m (J=84Hz).

E. (E)-1-Phthalimido-2-methyl-3-fluoro-2-propene

A solution of (*E*)-2-methyl-3-fluoroallyl alcohol (prepared in Step D) (17.11 g), triphenylphosphine (49.80 g), diethyl azodicarboxylate (33.06 g) and phthalimide (27.93 g) in THF (500 ml) is stirred at room temperature overnight. The THF is evaporated and the oily residue is extracted three times with hexane giving a powdery solid which is subsequently extracted three times with ether. The combined extracts are evaporated and the residue (63 g) is purified by chromatography on silica gel (950 g) using a mixture of 20% diethyl ether/light petroleum. The major fraction is a colorless crystalline mass (28.6 g, 69% yield) which is essentially pure product. A small portion can be crystallized from hexane go give *(E)-1-phthalimido-2-methyl-3-fluoro-2-propene* as colorless plates: m.p. 57—58°C.

NMR ($CDCl_3$): δ 1.67, d.d. (J=3.6Hz, 1.8Hz), 3H; 4.17, d (J=3.8Hz), 2H; 6.77, d.m (J=84Hz), 1H; centered at 7.82, m, 4H.

Analysis for $C_{12}H_{10}FNO_2$

| | | | |
|---|---|---|---|
| Found: | C, 65.71; | H, 4.75; | N, 6.26% |
| Requires: | C, 65.75: | H, 4.60; | N, 6.39% |

F. (Z)-1-Fluoro-2-bromomethyl-3-phthalimidopropene

A mixture of 1-phthalimido-2-methyl-3-fluoro-2-propene (prepared in Step E) 2.09 g) and N-bromosuccinamide (1.78 g) in carbon tetrachloride (100 ml) is refluxed for 45 min. The cooled mixture is filtered and the filtrate is washed with water, dried and evaporated to leave an almost colorless oil. Chromatography (silica; 20% ether in light petroleum) followed by recrystallization of the major fractions from EtOAc/light petroleum gives:

(a) The less polar (*Z*)-1-fluoro-2-bromomethyl-3-phthalimdo-propene (1.00 g; 35% yield) as colorless needles; mp. 81—83°C.

$C_{12}H_9BrFNO_2$

| | | | |
|---|---|---|---|
| Found: | C, 48.30; | H, 3.14; | N, 4.60% |
| Requires: | C, 48.34; | H, 3.04; | N 4.70% |

NMR ($CDCl_3$): δ 4.05, d (J=2Hz), 2H; 4.33, d(J=3Hz), 2H; 6.87, d (J=82Hz), 1H; 7.62 to 7.95, m, 4H.

(b) The more polar (*E*) 1-fluoro-2-bromomethyl-3-phthalimidopropene (0.25 g; 9% yield) as colorless needles; mp. 86—87°C.

$C_{12}H_9BrFNO_2$

| | | | |
|---|---|---|---|
| Found: | C, 48.39; | H, 3.14; | N, 4.66% |
| Requires: | C, 48.34; | H, 3.04; | N, 4.70% |

NMR ($CDCl_3$): δ 3.95, d (J=4Hz), 2H; 4.53, d.d (J=2.5Hz and less than 1Hz), 2H; 6.85, d (J=80Hz with additional fine coupling), 1H; 7.60 to 7.93, m, 4H.

G. (Z)-1-Fluoro-2-(2',4'-dichlorophenoxy)methyl-3-phthalimidopropene

Solid 1-fluoro-2-bromomethyl-3-phthalimidopropene (0.60 g) is added to a previously prepared mixture of 2,4-dichlorophenol (0.33 g) and sodium hydride dispersion (96 mg of 55—60% oil dispersion) in dimethylformamide (10 ml) at room temperature. Stirring is continued for 3 hours, then brine is added and the product is isolated by ether extraction. The extracted material is essentially pure (Z)-1-fluoro-(2',4'-dichlorophenoxy)methyl-3-phthalimidopropene (0.67 g; 88% yield). A small portion is recrystallized from hexane/dichloromethane whereupon the analytical sample is obtained as colorless plates; mp. 115—116°C.

$C_{18}H_{12}Cl_2FNO_3$

Found: C, 56.89; H, 3.25; N, 3.71%

Requires: C, 56.86; H, 3.18; N, 3.68%

NMR (CDCl$_3$): δ 4.37, d (J=3Hz), 2H; 4.70, d (J=2.5Hz), 2H; 6.80 to 7.23, m, 3H; 6.97, d (broadened, J=83Hz), 1H; 7.75, m, 4H.

H. (Z)-2-(2',4'-Dichlorophenoxy)methyl-3-fluorallylamine

A solution of (Z)-1-fluoro-2-(2',4'-dichlorophenoxy)methyl-3-phthalimidopropene (0.67 g) and hydrazine hydrate (0.13 g) in ethanol (20 ml) is refluxed for 3 hours. The ethanol is evaporated, the residue extracted with ether and the ether solution washed with dilute aqueous sodium hydroxide, then with water, dried and evaporated. The residue is treated with di-*tert*-butyl dicarbonate (0.44 g), chloroform (20 ml) and water (6 ml), with added sodium chloride (1 g), for 1.5 hours at reflux to give (Z)-N-*t*-butyloxycarbonyl-2-(2',4'-dichlorophenoxy)methyl-3-fluoroallylamine. Pure colorless needles (0.43 g) are obtained by silica chromatography using 15% EtOAC in light petroleum as eluant. Cleavage of the butyloxycarbonyl group (HCl/ether) gave (Z)-2-(2',4'-dichlorophenoxy)methyl-3-fluoroallylamine as its hydrochloride salt; colorless needles (0.30 g; 59% yield); mp 135—136°C.

$C_{10}H_{11}Cl_3FNO$

Found: C, 41.78; H, 4.02; N, 4.74%

Requires: C, 41.91; H, 3.87; N, 4.89%

NMR (CDCl$_3$): δ 3.35, d (J=4Hz), 2H; 4.80, d (J=2.5Hz), 2H; 5.97, m, ½H; 6.90, 7.18, 7.35, ABC system ($J_{AB}$=10Hz; $J_{BC}$=2Hz; $J_{AC}$ ~ OHz) overlapping 7.27, m, 3 ½H.

Example 2

The procedure of Steps G and H of Example 1 are repeated commencing with (E)-1-fluoro-2-bromomethyl-3-phthalimidopropene (prepared in Step F) instead of the (Z)-isomer to yield (E)-2-(2',4'-dichlorophenoxy)methyl-3-fluoroallylamine, m.p. 104°C.

Example 3

The procedure of Steps G and H of Example 1 are repeated using phenol instead of 2,4-dichlorophenol to yield (Z)-2-phenoxymethyl-3-fluoroallylamine m.p. 139—140°C.

Example 4

The procedure of Steps G and H of Example 1 are repeated using thiophenol instead of 2,4-dichlorophenol to yield (Z)-2-thiophenoxymethyl-3-fluoroallylamine, m.p. 164—165°C.

Example 5

The procedure of Steps G and H of Example 1 are repeated using *p*-fluorothiophenol instead of 2,4-dichlorophenol to yield the compound (Z)-2-(4'-fluorothiophenoxy)methyl-3-fluoroallylamine, m.p. 169°C.

Example 6

The procedure of Steps G and H of Example 2 are repeated using thiophenol instead of 2,4-dichlorophenol to yield the compound (E)-2-thiophenoxymethyl-3-fluoroallylamine, m.p. 128°C.

Example 7

N-Methyl (Z)-2-(2',4'-dichlorophenoxy)methyl-3-fluoroallylamine

A mixture of (Z)-*N*-*t*-butyloxycarbonyl-2-(2',4'-dichlorophenoxy)methyl-3-fluoroallylamine (550 mg), prepared as in Step H of Example 1, dissolved in dimethylformamide (10 ml) is treated with sodium hydride (37 mg) for 30 minutes. A solution of methyl iodide (223 mg) in dimethylformamide (5 ml) is slowly added and the reaction mixture stirred overnight. Ether extractions followed by silica chromatography yields the protected *N*-methyl derivative as a colorless oil (180 mg). This oil is dissolved in HCl/ether whereupon N-methyl (Z)-2-(2',4'-dichlorophenoxy)methyl-3-fluoroallylamine is obtained as colorless needles, m.p. 154°C.

Example 8

Inhibition of MAO — In vitro testing

(A) The ability of a compound of Formula I to inhibit MAO can be determined *in vitro* by the method of A. Christmas *et al., Br. J. Pharmacol., 45*, 490 (1972) in partially purified mitochondria from rat brain using

$^{14}C$ *p*-tyramine as the substrate. The MAO inhibitory activity of a compound is expressed as the "$IC_{50}$" value, which is the molar concentration required to produce 50% inhibition of the enzyme. The $IC_{50}$ values for certain compounds of Formula I were determined using the above-described method, and the results are set forth in Table I. For comparison, $IC_{50}$ values for clorgyline, L-deprenyl, and pargyline are also given. The data shown in Table I does not show selectivity of the compounds against MAO—A or MAO—B inhibitors, since $^{14}C$ *p*-tyramine is a substrate for both forms of the enzyme.

TABLE I
MAO Inhibitory Activity — In Vitro

| Compound (a) | $IC_{50}$ (moles) |
|---|---|
| (Z)-2-(2',4'-dichlorophenoxy)methyl-3-fluoroallylamine | $1.5 \times 10^{-7}$ |
| (Z)-2-phenoxymethyl-3-fluoroallylamine | $1 \times 10^{-6}$ |
| (Z)-2-thiophenoxymethyl-3-fluoroallylamine | $1 \times 10^{-6}$ |
| clorgyline | $1 \times 10^{-8}$ |
| L-deprenyl | $1 \times 10^{-7}$ |
| pargyline | $2 \times 10^{-6}$ |

(a) Tested as hydrochloride salt.

The data shown in Table I demonstrate that the compounds tested are potent inhibitors of MAO.

(B) The compounds of Formula I can be tested to determine whether or not the MAO inhibition follows time-dependent kinetics by the procedure described below:

Mitochondria are prepared from rat brain by homogenation in phosphate buffer (0.1 M, pH 7.2) followed by differential centrifugation. The mitochondria are suspended in the same buffer, the test compound is added at the desired concentration, and the system is incubated. At different time intervals, aliquots are taken and MAO activity is measured using $^{14}C$ *p*-tyramine (a mixed substrate) as the substrate (See A. Christmas *et al., supra*). When (Z)-2-(2',4'-dichlorophenoxy)methyl-3-fluoroallylamine was tested according to the above-described procedure, the MAO inhibitory activity increased as a function of time of incubation. The initial rate of decrease of activity increased with increasing concentration of inhibitor. The inhibition of MAO was shown to be irreversible since dialysis against phosphate buffer (24 hours) did not restore enzyme activity.

(C) The selectivity of a compound of Formula I with respect to inhibition of MAO—A and MAO—B can be determined by repeating the procedure of Part B and measuring the MAO activity against $^{14}C$ 5-hydroxyltryptamine (a preferred substrate for MAO—A) and $^{14}C$ phenethylamine (a preferred substrate for MAO—B) as the substrate. The selectivity is expressed as the ratio of the inhibitory activity against MAO—B versus the inhibitory activity against MAO—A. In the case of (Z)-2-(2',4'-dichlorophenoxy)methyl-3-fluoroallylamine said ratio is 200, i.e. the compound was 200 times more selective for MAO—B than for MAO—A. Other compounds tested have equivalent or better selectivity as shown in Table II below:—

TABLE II

| Compound | Ratio B:A |
|---|---|
| N-methyl (Z)-2-(2',4'-dichlorophenoxy)methyl-3-fluoroallylamine | 100 |
| (Z)-2-(4'-fluorothiophenoxy)methyl-3-fluoroallylamine | 100 |
| (E)-2-thiophenoxymethyl-3-fluoroallylamine | 100 |
| (Z)-2-thiophenoxymethyl-3-fluoroallylamine | 1,000 |

Example 9

Inhibition of MAO — Ex Vivo

The ability of a compound of Formula I to inhibit MAO can be determined *ex vivo* by the following procedure:

The test compound is administered orally (p.o.) to 300—350 g male Sprague-Dawley rats (Charles River, France) and the animals are killed 18 hours after treatment. The brain, heart, liver and/or duodenum is removed and either a crude homogenate or a mitochondrial fraction, described in Example 5, Part (A), is

prepared. MAO activity is determined in the homogenates using $^{14}C$ *p*-tyramine, as the substrate. Table III gives the results of the testing of (Z)-2-(2',4'-dichlorophenoxy)methyl-3-fluoroallylamine according to the above described procedure. Selectivity can be determined by repeating the above-described test using either $^{14}C$ *p*-hydroxytryptamine (for MAO—A) or $^{14}C$ phenethylamine (for MAO—B) as the substrate for determining the percentage inhibition.

TABLE III

| Dose (po) (mg/kg) | % inhibition Brain | Heart | Liver | Duodenum |
|---|---|---|---|---|
| | (1) (2) | (1) (2) | (1) (2) | (1) (2) |
| 1 | 41 85 | 27 40 | 0 57 | 10 73 |
| 2.5 | 23 90 | 0 31 | 9 67 | 26 82 |
| 5 | 34 93 | 0 40 | 21 89 | 55 95 |
| 10 | 55 96 | 0 40 | 25 90 | 57 96 |
| 25 | 73 98 | 32 64 | 55 95 | 65 98 |

(1) using $^{14}C$-p-tyramine as substrate
(2) using $^{14}C$-phenethylamine as substrate

It can be seen from Table III that the test compound produced preferential inhibition of MAO—B in the four tissues examined at the dose levels tested. Doses as low as 1 mg/kg p.o. produced greater than 80% inhibition of brain MAO—B with doses greater than 5 mg/kg being needed to inhibit MAO—A activity by more than 50%.

Example 10
Inhibition of MAO — In vivo

The ability of a compound of Formula I to inhibit MAO can be determined *in vivo* using brain and heart samples obtained from the *ex vivo* study reported in Example 9. Monoamines and their deaminated metabolites were determined by HPLC with electrochemical detection by the method of J. Wagner *et al,* J. Neurochem. *38* 1241—1254.

When (Z)-2-(2',4'-dichlorophenoxy)-methyl-2-fluoroallylamine is tested according to the above-described procedure, the results given in Tables IV and V are obtained. In these tables the following abbreviations are used.

DA = dopamine,

HVA = homovanillic acid

NE = norepinephrine

DA = dopamine

DOPAC = dihydroxphenylacetic acid

5—HT = 5-hydroxytryptamine

5—HIAA = 5-hydroxyindole-3-acetic acid

TABLE IV

EFFECT ADMINISTERED P.O. 18 HRS PREVIOUSLY ON BRAIN MONOAMINES AND THEIR METABOLITES.

| | DA | DOPAC | HVA | NE | 5—HT | 5HIAA |
|---|---|---|---|---|---|---|
| | | | ng/g S.E.M. | | | |
| Control (saline) | 840 ± 27 | 80 ± 3 | 81 ± 3 | 369 ± 18 | 1156 ± 52 | 347 ± 8 |
| 1 mg/kg | 945 ± 35 | 75 ± 11 | 94 ± 15 | 386 ± 18 | 1218 ± 66 | 371 ± 19 |
| 2.5 mg/kg | 896 ± 31 | 70 ± 6 | 75 ± 7 | 409 ± 4 | 1119 ± 64 | 337 ± 15 |
| | 943 ± 21 | 65 ± 3 | 83 ± 7.2 | 415 ± 18 | 1340 ± 116 | 379 ± 10 |
| 5 mg/kg | (<0.02) | (<0.01) | | | | (<0.05) |
| 10 mg/kg | 875 ± 16 | ±3 (<0.005) | 53 ± 10 (<0.02) | 439 ± 15 (<0.02) | 1297 ± 100 | 343 ± 14 |
| 25 mg/kg | 1087 ± 29 (<0.001) | 54 ± 6 (<0.01) | 75 ± 8 | 496 ± 12 (<0.001) | 1623 ± 48 (<0.001) | 404 ± 12 (<0.005) |

p values indicating that individual values differ from the relevant control values are presented in brackets.

TABLE V
EFFECT ADMINISTERED P.O. 18 HRS PREVIOUSLY ON BRAIN MONOAMINES AND THEIR METABOLITES.

| | NE | DOPAC | HVA | 5HT | 5HIAA |
|---|---|---|---|---|---|
| Control | 735 ± 100 | 78 ± 22 | 38 ± 9 | 808 ± 50 | 7 ± 1 |
| 1 mg/kg | 741 ± 56 | 56 ± 11 | 71 ± 6 (<0.02) | 1017 ± 79 | 16 ± 4 |
| 2.5 mg/kg | 762 ± 20 | 46 ± 12 | 53 ± 8 | 995 ± 108 | 7 ± 1 |
| 5 mg/kg | 875 ± 53 | 56 ± 7 | | | |
| 10 mg/kg | 729 ± 58 | 62 ± 5 | 55 ± 3 | 850 ± 82 | 14 ± 2 |
| 25 mg/kg | 765 ± 50 | 87 ± 27 | 65 ± 4 (<0.02) | 685 ± 113 | 26 ± 4 (<0.02) |

p values indicating that individual values differ from the relevant control values are presented in brackets.

As can be seen from Table IV significant reductions in dihydroxyphenylacetic acid and increases in dopamine were evident in the brain following the 5 mg/kg dose. Norepinephrine (NE) concentrations were increased significantly at the 10 and 25 mg/kg dose. Significant increases in 5—HT were obtained at the 25 mg/kg dose. However, as seen from Table V, no consistent changes in monoamines or their metabolites were obtained in the heart. These data are consistent with selectivity of action against MAO—B at the lower doses and a small degree of MAO—A inhibition occurring at the higher doses of the test compound.

Example 11

The following test procedures can be employed to assess the potential of a compound of Formula I for producing the "cheese effect":

Male Spraque-Dawley rats (Charles River, France) weighing 240—347 g are given single doses of either 5, 10 or 25 mg/kg of the test compound by mouth. Eighteen hours later the animals are anaesthetized with pentobarbitone (60 mg/kg i.p.), in some instances pithed, and in all cases set up for recording heart rate and blood pressure by standard techniques. The effects of the test compound on intravenous tyramine were estimated in pithed rats using incremental doses of tyramine from 1.25 to 80 µg/kg injected every 7 minutes into a cannulated femoral vein. The effects on intraduodenal tyramine were assessed in anaesthetized rats by administering doses between 0.312 and 50 mg/kg at intervals of 15 min via a cannula placed in the duodenum. The results obtained for (Z)-2-(2′,4′-dichlorophenoxy)methyl-3-fluoroallylamine are set forth in Table V.

As can be seen from Table VI, cardiovascular responses to tyramine injected intravenously were affected to only a small extent by 10 mg/kg given p.o. 18 h prior to testing. In two experiments, a clear 2—3 fold potentiation of tyramine was obtained following treatment with 25 mg/kg.

TABLE VI
Potentiation of heart rate response to p-tyramine

| Dose mg/kg | Route of administration of p-tyramine | Potentation of heart rate response to p-tyramine |
|---|---|---|
| (Z)-2-(2′,4′-dichlorophenoxy)methyl-3-fluoroallylamine | | |
| 5 | i.d. | 1.5 fold |
| 10 | i.v. | none |
| 10 | i.d. | 2.4 fold |
| 25 | i.v. | 2.8 fold |
| 25 | i.d. | 2.0 fold |
| L-deprenyl | | |
| 0.1 | i.v. | 1.3 fold |
| 1.0 | i.v. | 2.2 fold |
| 0.1 | i.d. | no effect |
| 1.0 | i.d. | 2.1 fold |
| clorgyline | | |
| 0.1 | i.v. | 5.2 fold |
| 0.1 | i.d. | 5.6 fold |

i.v.: tyramine administered intravenously
i.d.: tyramine administered intraduodenally

In the following Examples relating to pharmaceutical compositions, the term "active compound" is used to indicate the compound (Z)-2-(2′,4′-dichlorophenoxy)methyl-3-fluoroallylamine. This compound may be replaced in these compositions by any other compound of the invention. Adjustments in the amount of medicament may be necessary or desirable depending upon the degree of activity of the medicament as is well known in the art.

Example 12

An illustration composition of hard gelatin capsules is as follows:

| | | |
|---|---|---|
| (a) | Active compound | 5 mg |
| (b) | Talc | 5 mg |
| (c) | Lactose | 90 mg |

The formulation is prepared by passing the dry powders of (a) and (b) through a fine mesh screen and mixing them well. The powder is then filled into hard gelatine capsules at a net fill of 100 mg per capsule.

Example 13

An illustrative composition for tablets is as follows:

| | | |
|---|---|---|
| (a) | Active compound | 5 mg |
| (b) | Starch | 45 mg |
| (c) | Lactose | 48 mg |
| (d) | Magnesium stearate | 2 mg |

The granulation obtained upon mixing the lactose with the compound (a) and the part of the starch and granulated with starch paste is dried, screened, and mixed with the magnesium stearate. The mixture is compressed into tablets weighing 100 mg each.

Example 14

An illustrative composition for an injectable suspension is the following 1 ml ampule for an intramuscular injection.

| | Weight per cent |
|---|---|
| (a) Active compound | 0.5 |
| (b) Polyvinylpyrrolidone | 0.5 |
| (c) Lecithin | 0.25 |
| (d) Water fron injection to make | 100.00 |

The materials (a)—(d) are mixed, homogenized, and filled into 1 ml ampule which are sealed and autoclaved 20 minutes at 121°C. Each ampule contains 5 mg per mol of the active compound.

**Claims for the Contracting States: BE CH DE FR IT LU NL SE LI**

1. A fluoroallylamine derivative of the following Formula I:

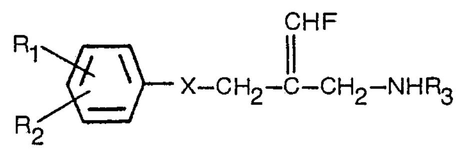

(I)

wherein:

$R_1$ and $R_2$ independently represent hydrogen, chlorine or fluorine;
$R_3$ represents hydrogen or ($C_1$—$C_4$) alkyl; and
X represents oxygen or sulfur,

or a pharmacologically acceptable acid addition salt thereof.

2. A compound as claimed in Claim 1, wherein $R_3$ represents hydrogen.

3. A compound as claimed in Claim 1, wherein $R_3$ represents methyl or ethyl.

4. A compound as claimed in any one of the preceding claims, wherein $R_1$ and $R_2$ are both hydrogen.

5. A compound as claimed in any one of Claims 1 to 3, wherein $R_1$ represents hydrogen and $R_2$ represents chlorine or fluorine.

6. A compound as claimed in any one of Claims 1 to 3, wherein $R_1$ and $R_2$ independently represent chlorine or fluorine.

7. A compound as claimed in Claim 6, wherein the phenyl ring is 2,4-disubstituted by $R_1$ and $R_2$.

8. A compound as claimed in any one of the preceding claims, wherein X represents oxygen.

9. A compound as claimed in any one of Claims 1 to 7, wherein X represents sulfur.

10. A compound selected from:
(a) (Z)-2-phenoxymethyl-3-fluoroallylamine;
(b) (Z)-2-thiophenoxymethyl-3-fluoroallylamine;
(c) (E)-2-thiophenoxymethyl-3-fluoroallylamine;
(d) (Z)-2-(2′,4′-dichlorophenoxy)methyl-3-fluoroallylamine;
(e) (E)-2-(2′,4′-dichlorophenoxy)methyl-3-fluoroallylamine;
(f) (Z)-2-(4′-fluorothiophenoxy)methyl-3-fluoroallylamine
(g) N-methyl-(Z)-2-(2′,4′-dichlorophenoxy)-methyl-3-fluoroallylamine;
or a pharmaceutically acceptable acid addition salt thereof.

11. A compound as claimed in any one of Claims 1 to 10 for use as a medicine.

12. A compound as claimed in any one of Claims 1 to 10, for use in the inhibition of monoamine oxidas in a patient.

13. A compound as claimed in any one of Claims 1 to 10, for use in the treatment of depression.

14. The use of a compound as claimed in any one of Claims 1 to 10, for the manufacture of a medicament for the inhibition of monoamine oxidase in a patient.

15. The use of a compound as claimed in any one of claims 1 to 10, for the manufacture of a medicament for the treatment of depression.

16. A pharmaceutical composition comprising a compound as claimed in any one of Claims 1 to 10 in admixture or otherwise associated with a pharmaceutically acceptable carrier or diluent.

17. A process for preparing a compound as claimed in claim 1 which comprises reacting an amino-protected derivative of 1-fluoro-2-bromomethyl-3-aminopropene of formula II

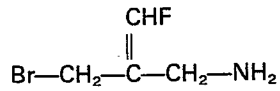

(II)

with a phenol or thiophenol of the following formula III

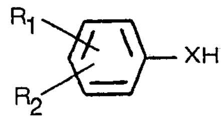

(III)

wherein X, $R_1$ and $R_2$ are as defined in claim 1, in an aprotic solvent under anhydrous conditions in the presence of a strong base, subsequently removing the amino-protecting group and, when a compound of claim 1 wherein $R_3$ is $(C_1—C_4)$alkyl is desired, submitting the obtained compound wherein $R_3$ is hydrogen to N-alkylation.

**Claims for the Contracting State: AT**

1. A process for preparing a fluoroallylamine derivative of the following formula I:

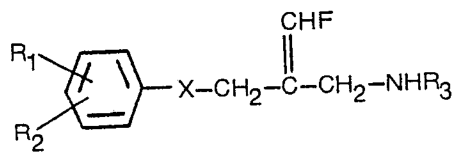

(I)

wherein:
$R_1$ and $R_2$ independently represent hydrogen, chlorine or fluorine;
$R_3$ represents hydrogen or $(C_1—C_4)$ alkyl; and X represents oxygen or sulfur,
or a pharmacologically acceptable acid addition salt thereof, which comprises reacting an amino-protected derivative of 1-fluoro-2-bromomethyl-3-aminopropene of formula II:

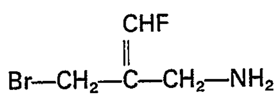

(II)

with a phenol or thiophenol of the following formula III:

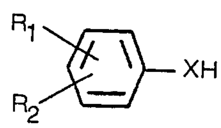

(III)

wherein X, $R_1$ and $R_2$ are as defined above, in an aprotic solvent under anhydrous conditions in the presence of a strong base, subsequently removing the amino-protecting group and, when a compound of claim 1 wherein $R_3$ is $(C_1—C_4)$alkyl is desired, submitting the obtained compound wherein $R_3$ is hydrogen to N-alkylation.

2. A process as claimed in claim 1 for preparing a compound of formula I wherein $R_3$ represents hydrogen.

3. A process as claimed in claim 1 for preparing a compound of formula I wherein $R_3$ represents methyl or ethyl.

4. A process as claimed in claim 1 for preparing a compound of formula I wherein $R_1$ and $R_2$ are both hydrogen.

5. A process as claimed in claim 1 for preparing a compound of formula I wherein $R_1$ represents hydrogen and $R_2$ represents chlorine or fluorine.

6. A process as claimed in claim 1, 2 or 3 for preparing compound of formula I wherein $R_1$ and $R_2$ independently represent chlorine or fluorine.

7. A process as claimed in claim 1, 2 or 3 for preparing a compound of formula I wherein the phenyl ring is 2,4-disubstituted by $R_1$ and $R_2$.

8. A process as claimed in claim 1 for preparing a compound of formula I wherein X represents oxygen.

9. A process as claimed in claim 1 for preparing a compound of formula I wherein X represents sulfur.

10. A process as claimed in claim 1, for preparing a compound selected from:
(a) (Z)-2-phenoxymethyl-3-fluoroallylamine;
(b) (Z)-2-thiophenoxymethyl-3-fluoroallylamine;

(c) (E)-2-thiophenoxymethyl-3-fluoroallylamine;
(d) (Z)-2-(2′,4′-dichlorophenoxy)methyl-3-fluoroallylamine;
(e) (E)-2-(2′,4′-dichlorophenoxy)methyl-3-fluoroallylamine;
(f) (Z)-2-(4′-fluorothiophenoxy)methyl-3-fluoroallylamine
(g) N-methyl-(Z)-2-(2′,4′-dichlorophenoxy)-methyl-3-fluoroallylamine;
or a pharmaceutically acceptable acid addition salt thereof.

11. A process as claimed in claim 1 wherein the reaction between amino-protected derivative of 1-fluoro-2-bromomethyl-3-aminopropene of formula II and the phenol or thiophenol of formula III is conducted at room temperature.

12. A process as claimed in claim 1 wherein the strong base is sodium hydride or butyl lithium.

13. A process as claimed in claim 1 wherein the protecting group of the amino function of 1-fluoro-2-bromomethyl-3-aminopropene of formula II is a phthaloyl group.

**Patentansprüche für die Vertragsstaaten: BE CH LI DE FR IT LU NL SE**

1. Ein Fluorallylaminderivat der folgenden Formel I:

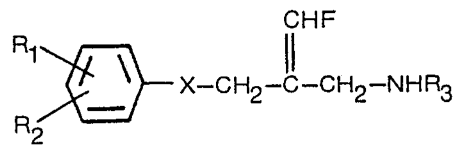

(I)

worin
$R_1$ und $R_2$ unabhängig voneinander Wasserstoff, Chlor oder fluor bedeuten;
$R_3$ Wasserstoff oder ($C_1$—$C_4$) Alkyl bedeutet und X Sauerstoff oder Schwefel bedeutet oder ein pharmakologisch verträgliches Säureadditionssalz davon.

2. Eine Verbindung nach Anspruch 1, worin $R_3$ Wasserstoff bedeutet.

3. Eine Verbindung nach Anspruch 1, worin $R_3$ Methyl oder Ethyl bedeutet.

4. Eine Verbindung nach einem der vorangehenden Ansprüche, worin $R_1$ und $R_2$ jeweils Wasserstoff bedeuten.

5. Eine Verbindung nach einem der Ansprüche 1 bis 3, worin $R_1$ Wasserstoff und $R_2$ Chlor oder Fluor bedeutet.

6. Eine Verbindung nach einem der Ansprüche 1 bis 3, worin $R_1$ und $R_2$ unabhängig voneinander Chlor oder Fluor bedeuten.

7. Eine Verbindung nach Anspruch 6, worin der Phenylring durch $R_1$ und $R_2$ 2,4-disubstituiert ist.

8. Eine Verbindung nach einem der vorangehenden Ansprüche, worin X Sauerstoff bedeutet.

9. Eine Verbindung nach einem der Ansprüche 1 bis 7, worin X Schwefel bedeutet.

10. Eine Verbindung gewählt aus:
(a) (Z)-2-Phenoxymethyl-3-fluorallylamin;
(b) (Z)-2-Thiophenoxymethyl-3-fluorallylamin;
(c) (E)-2-Thiophenoxymethyl-3-fluorallylamin;
(d) (Z)-2-(2′,4′-Dichlorphenoxy)methyl-3-fluorallylamin;
(e) (E)-2-(2′,4′-Dichlorphenoxy)methyl-3-fluorallylamin;
(f) (Z)-2-(4′-Fluorthiophenoxy)methyl-3-fluorallylamin;
(g) N-Methyl-(Z)-2-(2′,4′-dichlorphenoxy)-methyl-3-fluorallylamin;
oder ein pharmazeutisch verträgliches Säureadditionssalz davon.

11. Eine Verbindung nach einem der Ansprüche 1 bis 10 zur Verwendung als Medikament.

12. Eine Verbindung nach einem der Ansprüche 1 bis 10 zur Verwendung der Inhibierung der Monoaminoxidase bei einem Patienten.

13. Eine Verbindung nach einem der Ansprüche 1 bis 10 zur Verwendung bei der Depressionsbehandlung.

14. Die Verwendung einer Verbindung nach einem der Ansprüche 1 bis 10, zur Herstellung eines Medikaments zur Inhibierung der Monoaminoxidase bei einem Patienten.

15. Die Verwendung einer Verbindung nach einem der Ansprüche 1 bis 10, zur Herstellung eines Medikaments für die Depressionsbehandlung.

16. Eine pharmazeutische Zubereitung, welche eine Verbindung nach einem der Ansprüche 1 bis 10 in Mischung oder anderer Verbindung mit einem pharmazeutisch verträglichen Träger oder Verdünnungsmittel enthält.

17. Ein Verfahren zur Herstellung einer Verbindung nach Anspruch 1, dadurch gekennzeichnet, daß ein an der Aminogruppe geschütztes 1-Fluor-2-brommethyl-3-aminopropen-Derivat der Formel II

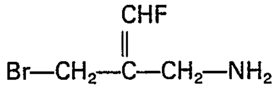

(II)

mit einem Phenol oder Thiophenol der folgenden Formel III

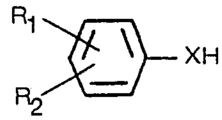

(III)

worin X, $R_1$ und $R_2$ wie in Anspruch 1 definiert sind, in einem aprotischen Lösungsmittel unter wasserfreien Bedingungen in Gegenwart einer starken Base umgesetzt wird, anschließend die Amino-Schutzgruppe entfernt wird und wenn eine Verbindung nach Anspruch 1 gewünscht wird, in welcher $R_3$ ($C_1$—$C_4$) Alkyl bedeutet, wird die erhaltene Verbindung, in welcher $R_3$ Wasserstoff bedeutet, einer N-Alkylierung unterworfen.

**Patentansprüche für den Vertragsstaat: AT**

1. Ein Verfahren zur Herstellung eines Fluorallylaminderivates der folgenden Formel I:

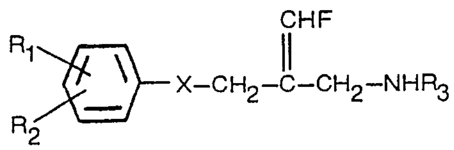

(I)

worin
$R_1$ und $R_2$ unabhängig voneinander Wasserstoff, Chlor oder Fluor bedeuten;
$R_3$ Wasserstoff oder ($C_1$—$C_4$) Alkyl bedeutet und X Sauerstoff oder Schwefel bedeutet oder ein pharmakologisch verträgliches Säureadditionssalz davon, dadurch gekennzeichnet, daß ein an der Aminogruppe geschütztes 1-Fluor-2-brommethyl-3-aminopropen-Derivat der Formel II

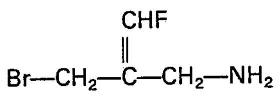

(II)

mit einem Phenol oder Thiophenol der folgenden Formel III

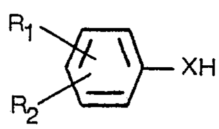

(III)

worin X, $R_1$ und $R_2$ wie oben definiert sind, in einem aprotischen Lösungsmittel unter wasserfreien Bedingungen in Gegenwart einer starken Base umgesetzt wird, anschließend die Amino-Schutzgruppe entfernt wird und wenn eine Verbindung der Formel I gewüscht wird, in welcher $R_3$ ($C_1$—$C_4$) Alkyl bedeutet, wird die erhaltene Verbindung, in welcher $R_3$ Wasserstoff bedeutet, einer N-Alkylierung unterworfen.

2. Ein Verfahren nach Anspruch 1, zur Herstellung einer Verbindung der Formel I, worin $R_3$ Wasserstoff bedeutet.

3. Ein Verfahren nach Anspruch 1, zur Herstellung einer Verbindung der Formel I, worin $R_3$ Methyl oder Ethyl bedeutet.

4. Ein Verfahren nach Anspruch 1, zur Herstellung einer Verbindung der Formel I, worin $R_1$ und $R_2$ jeweils Wasserstoff bedeuten.

5. Ein Verfahren nach Anspruch 1, zur Herstellung einer Verbindung der Formel I, worin $R_1$ Wasserstoff und $R_2$ Chlor oder Fluor bedeutet.

6. Ein Verfahren nach Anspruch 1, 2 oder 3, zur Herstellung einer Verbindung der Formel I, worin $R_1$ und $R_2$ unabhängig voneinander Chlor oder Fluor bedeuten.

7. Ein Verfahren nach Anspruch 1, 2 oder 3, zur Herstellung einer Verbindung der Formel I, worin der Phenylring durch $R_1$ und $R_2$ 2,4-disubstituiert ist.

8. Ein Verfahren nach Anspruch 1, zur Herstellung einer Verbindung der Formel I, worin X Sauerstoff bedeutet.

9. Ein Verfahren nach Anspruch 1, zur Herstellung einer Verbindung der Formel I, worin X Schwefel bedeutet.

10. Ein Verfahren nach Anspruch 1, zur Herstellung einer Verbindung gewählt aus
(a) (Z)-2-Phenoxymethyl-3-fluorallylamin;
(b) (Z)-2-Thiophenoxymethyl-3-fluorallylamin;
(c) (E)-2-Thiophenoxymethyl-3-fluorallylamin;
(d) (Z)-2-(2′,4′-Dichlorphenoxy)methyl-3-fluorallylamin;
(e) (E)-2-(2′,4′-Dichlorphenoxy)methyl-3-fluorallylamin;

(f) (Z)-2-(4'-Fluorthiophenoxy)methyl-3-fluorallylamin;

(g) N-Methyl-(Z)-2-(2',4'-dichlorphenoxy)-methyl-3-fluorallylamin;

oder ein pharmazeutisch verträgliches Säureadditionssalz davon.

11. Ein Verfahren nach Anspruch 1, worin die Reaktion zwischen dem an der Aminogruppe geschützten 1-Fluor-2-brommethyl-3-aminopropen-Derivat der Formel II und dem Phenol oder Thiophenol der Formel III bei Raumtemperatur durchgeführt wird.

12. Ein Verfahren nach Anspruch 1, worin die starke Base Natriumhydrid oder Butyllithium ist.

13. Ein Verfahren nach Anspruch 1, worin die Schutzgruppe der Aminofunktion des 1-Fluor-2-brommethyl-3-aminopropens der Formel II eine Phthaloylgruppe ist.

**Revendications pour les Etats contractants: BE CH LI DE FR IT LU NL SE**

1. Un dérivé de fluorallylamine de formule I suivante:

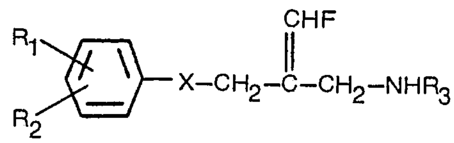

(I)

dans laquelle:

$R_1$ et $R_2$ représentent indépendamment l'hydrogène, le chlore ou le fluor,

$R_3$ représente l'hydrogène ou alkyle en $C_1$—$C_4$; et

X représente l'oxygène ou le soufre,

ou un de ses sels d'addition d'acides acceptables en pharmacie.

2. Un composé selon la revendication 1, dans lequel $R_3$ représente l'hydrogène.

3. Un composé selon la revendication 1, dans lequel $R_3$ représente méthyle ou éthyle.

4. Un composé selon l'une quelconque des revendications précédentes, dans lequel $R_1$ et $R_2$ sont tous deux l'hydrogène.

5. Un composé selon l'une quelconque des revendications 1 à 3, dans lequel $R_1$ représente l'hydrogène et $R_2$ représente le chlore ou le fluor.

6. Un composé selon l'une quelconque des revendications 1 à 3, dans lequel $R_1$ et $R_2$ représentent indépendamment le chlore ou le fluor.

7. Un composé selon la revendication 6, dans lequel le noyau phényle est 2,4-disubstitué par $R_1$ et $R_2$.

8. Un composé selon l'une quelconque des revendications précédentes, dans lequel X représente l'oxygène.

9. Un composé selon l'une quelconque des revendications 1 à 7, dans lequel X représente le soufre.

10. Un composé choisi parmi les suivants:

(a) (Z)-2-phénoxyméthyl-3-fluoroallylamine;

(b) (Z)-2-thiophénoxyméthyl-3-fluoroallylamine;

(c) (E)-2-thiophénoxyméthyl-3-fluoroallylamine;

(d) (Z)-2-(2',4'-dichlorophénoxy)méthyl-3-fluoroallylamine;

(e) (E)-2-(2',4'-dichlorophénoxy)méthyl-3-fluoroallylamine;

(f) (Z)-2-(4'-fluorothiophénoxy)méthyl-3-fluoroallylamine;

(g) N-méthyl-(Z)-2-(2',4'-dichlorophénoxy)méthyl-3-fluoroallylamine;

ou un de ses sels d'addition d'acides acceptables en pharmacie.

11. Un composé selon l'une quelconque des revendications 1 à 10 pour l'utilisation comme médicament.

12. Un composé selon l'une quelconque des revendications 1 à 10 pour l'utilisation dans l'inhibition de la monoamine-oxydase chez un patient.

13. Un composé selon l'une quelconque des revendications 1 à 10 pour l'utilisation dans le traitement de la dépression.

14. L'utilisation d'un composé selon l'une quelconque des revendications 1 à 10 pour la fabrication d'un médicament pour l'inhibition de la monoamine-oxydase chez un patient.

15. L'utilisation d'un composé selon l'une quelconque des revendications 1 a` 10 pour pour la fabrication d'un médicament pour le traitement de la dépression.

16. Une composition pharmaceutique comprenant un composé selon l'une quelconque des revendications 1 à 10 en mélange ou autre association avec un support ou diluant acceptable en pharmacie.

17. Un procédé pour fabriquer un composé selon la revendication 1, qui consiste à faire réagir un dérivé amino-protégé de 1-fluoro-2-bromométhyl-3-aminopropène de formule II:

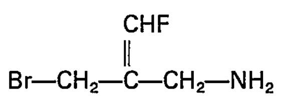

(II)

avec un phénol ou thiophénol de formule III suivante:

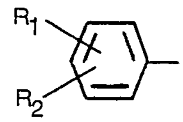

(III)

dans laquelle X, $R_1$ et $R_2$ sont tels que définis à la revendication 1, dans un solvant aprotique en conditions anhydres en présence d'une base forte, ensuite à éliminer le groupement amino-protecteur et, lorsque l'on désire un composé selon la revendication 1, dans lequel $R_3$ est un groupe alkyle en $C_1$—$C_4$, à soumettre le composé obtenu dans lequel $R_3$ est l'hydrogène à la N-alkylation.

**Revendications pour l'Etat contractant: AT**

1. Un procédé pour fabriquer un dérivé de fluoroallylamine de formule I suivante:

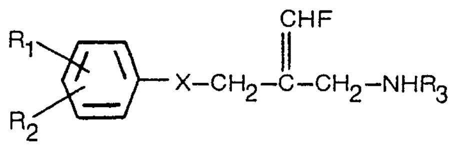

(I)

dans laquelle:
$R_1$ et $R_2$ représentent indépendamment l'hydrogène, le chlore ou le fluor,
$R_3$ représente l'hydrogène ou alkyle en $C_1$—$C_4$; et
X représente l'oxygène ou le soufre,
ou un de ses sels d'addition d'acides acceptables en pharmacie, qui consiste à faire réagir un dérivé amino-protégé de 1-fluoro-2-bromométhyl-3-aminopropène de formule II:

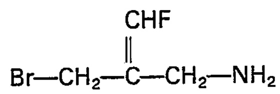

(II)

avec un phénol ou thiophénol de formule III suivante:

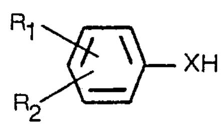

(III)

dans laquelle X, $R_1$ et $R_2$ sont tels que définis ci-dessus, dans un solvant aprotique en conditions anhydres, en présente d'une base forte, ensuite à éliminer le groupement amino-protecteur et, lorsque l'on désire un composé selon la revendication 1 dans lequel $R_3$ est un groupe alkyle en $C_1$—$C_4$, à soumettre le composé obtenu dans lequel $R_3$ est l'hydrogène à la N-alkylation.

2. Un procédé selon la revendication 1 pour fabriquer un composé de formule I, dans laquelle $R_3$ représente l'hydrogène.

3. Un procédé selon la revendication 1 pour fabriquer un composé de formule I, dans laquelle $R_3$ représente méthyle ou éthyle.

4. Un procédé selon la revendication 1 pour fabriquer un composé de formule I, dans laquelle $R_1$ et $R_2$ sont tous deux l'hydrogène.

5. Un procédé selon la revendication 1 pour fabriquer un composé de formule I, dans laquelle $R_1$ représente l'hydrogène et $R_2$ représente le chlore ou le fluor.

6. Un procédé selon la revendication 1, 2 ou 3 pour fabriquer un composé de formule I, dans laquelle $R_1$ et $R_2$ représentent indépendamment le chlore ou le fluor.

7. Un procédé selon la revendication 1, 2 ou 3 pour fabriquer un composé de formule I, dans laquelle le noyau phényle est 2,4-disubstitué par $R_1$ et $R_2$.

8. Un procédé selon la revendication 1 pour fabriquer un composé de formule I dans laquelle X représente l'oxygène.

9. Un procédé selon la revendication 1 pour fabriquer un composé de formule I dans laquelle X représente le soufre.

10. Un procédé selon la revendication 1, pour fabriquer un composé choisi parmi les suivants:
(a) (Z)-2-phénoxyméthyl-3-fluoroallylamine;
(b) (Z)-2-thiophénoxyméthyl-3-fluoroallylamine;
(c) (E)-2-thiophénoxyméthyl-3-fluoroallylamine;
(d) (Z)-2-(2',4'-dichlorophénoxy)méthyl-3-fluoroallylamine;

(e) (E)-2-(2′,4′-dichlorophénoxy)méthyl-3-fluoroallylamine;

(f) (Z)-2-(4′-fluorothiophénoxy)méthyl-3-fluoroallylamine;

(g) N-méthyl-(Z)-2-(2′,4′-dichlorphénoxy)méthyl-3-fluoroallylamine;

ou un de ses sels d'addition d'acides acceptables en pharmacie.

11. Un procédé selon la revendication 1, dans lequel la réaction entre le dérivé amino-protégé de 1-fluoro-2-bromométhyl-3-aminopropène de formule II et le phénol ou thiophénol de formule III est effectuée à la température ambiante.

12. Un procédé selon la revendication 1, dans lequel la base forte est l'hydrure de sodium ou le butyllithium.

13. Un procédé selon la revendication 1, dans lequel le groupe protecteur de la fonction amino du 1-fluoro-2-bromométhyl-3-aminopropène est un groupe phtaloyle.